# FASCICULE DE BREVET EUROPEEN

(11) **EP 1 070 506 B1**
(45) Date de publication et mention de la délivrance du brevet: **06.10.2004**
(21) Numéro de dépôt: 00401988.1
(22) Date de dépôt: 10.07.2000
(51) Int. Cl.: A61L 2/18, A61B 1/12

(54) **Désinfection d'endoscopes avec une solution aqueuse d'acide peracétique et de peroxyde d'hydrogène**
Desinfektion von Endoskopen mit einer wässrigen Lösung aus Peressigsäure und Wasserstoffperoxid
Disinfection of endoscopes with an aqueous solution of peracetic acid and hydrogen peroxide

(30) Priorité: 12.07.1999 FR 9909015
(43) Date de publication de la demande: 24.01.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Gamet, Jean Claude, 71460 Saint Martin du Tartre (FR); Gouges, Yves, 75014 Paris (FR); Le Rouzic, Daniel, 95120 Ermont (FR)
(74) Mandataire: Conan, Philippe Claude

(56) Documents cités:
- EP-A- 0 658 309
- EP-A- 0 873 687
- WO-A-94/15465
- US-A- 5 425 815

## Description

L'invention a pour objet un nouveau procédé de désinfection des endoscopes mis en oeuvre dans des machines à désinfecter ou dans des machines à laver et à désinfecter des endoscopes.

L'évolution actuelle des soins médicaux conduit à la multiplication des techniques exploratrices mettant en oeuvre des dispositifs de plus en plus petits, souvent fabriqués avec des matériaux thermosensibles. Il s'ensuit que ces dispositifs ne peuvent pas être stérilisés à chaud et que, lorsqu'ils ne sont pas à usage unique, ils doivent être nettoyés et désinfectés chimiquement après chaque utilisation. Selon la nature du tissu avec lequel le dispositif médical entre en contact lors de son utilisation, on établit trois niveaux de risques infectieux, auxquels correspondent des niveaux de traitement de désinfection requis pour un spectre d'activité à atteindre. Au haut niveau de risque infectieux, correspond un traitement de haut niveau qui doit être efficace contre tous les types microorganismes y compris les spores bactériennes. A titre d'exemples de matériels qui doivent subir ce traitement de haut niveau, à défaut de pouvoir subir une stérilisation à chaud, on peut citer les instrumentations et moteurs de microchirurgie et coeliochirurgie, les pièces à mains en dentisterie, en stomatologie et en chirurgie, le matériel bio-feedback en urologie et de nombreux endoscopes.

Une opération de désinfection chimique comprend généralement les étapes suivantes :
a) un pré-traitement, dont le but consiste à faciliter les étapes ultérieures en empêchant par exemple les salissures de sécher ; c'est ainsi qu'à la fin de leur utilisation les canaux des instruments médicaux en comportant, sont au plus tôt irrigués et/ou immergés dans une solution détergente éventuellement bactéricide ;
b) un nettoyage, dont l'objectif est d'éliminer les salissures, qui va conjuguer l'action physico-chimique de la solution utilisée et les actions mécaniques effectuées manuellement ou par des machines ;
c) la désinfection chimique, impliquant la mise en contact du dispositif à désinfecter avec une solution désinfectante, peut se faire soit par un procédé manuel, soit par un procédé semi-automatique ou automatique ; un procédé automatique met en oeuvre soit des appareils spécifiques pour la désinfection de contenants, d'endoscopes ou d'instrumentation, soit des aérolyseurs pour la désinfection par voie aérienne des surfaces de locaux et des équipements qu'ils renferment ;
d) le rinçage final, dont l'objectif est d'éliminer tout résidu de produit, tout en évitant les re-contaminations ; ceci implique l'utilisation d'une eau adaptée à cette exigence qui, selon le niveau de risque, sera stérile ou filtrée ; et, lorsque le dispositif n'est pas immédiatement réutilisé,
e) le séchage, puis
f) le stockage, ces deux dernières étapes devant, elles aussi, être effectuées dans des conditions évitant les re-contaminations du dispositif.

Dans la désinfection automatique des endoscopes, les étapes b) à e) sont réalisées dans des machines spécifiques à ces instruments. De tels appareils sont censés apporter une meilleure fiabilité, tant en termes de traçabilité que de reproductibilité du traitement ; certains d'entre eux ne réalisent que l'étape de désinfection, mais la plupart effectuent à la fois le nettoyage, la désinfection le rinçage final et le séchage. On peut citer par exemple, la machine pour le lavage et la désinfection des endoscopes nommée "Fibro-Cleaner", et commercialisée par la société LANCER qui effectue automatiquement en 30 minutes une opération de lavage désinfection d'un seul ou de deux endoscopes en même temps selon le processus en 8 phases suivant :

| Phases | Fonctions | Durée en minutes | Température °C | Produits |
|---|---|---|---|---|
| 0 | Rinçage | 0,5 | 25 | eau filtrée |
| 1 | Lavage | 2 | 25 | Détergent D.L.C. à 0,5% |
| 2 | Rinçage | 0,5 | 25 | eau filtrée |
| 3 | Rinçage | 0,5 | 25 | eau filtrée |
| 4 | Désinfection | 10 | 25 | glutaraldéhyde à 2% |
| 5 | Rinçage | 0,5 | 25 | eau filtrée |
| 6 | Rinçage | 0,5 | 25 | eau filtrée |
| 8 | Soufflage | 2 | - | air filtré |

L'étape de désinfection est mise en oeuvre avec une composition désinfectante, généralement concentrée en principes actifs, que la machine à laver et à désinfecter les endoscopes, dilue automatiquement à la concentration efficace. La température, et le temps de désinfection, sont des paramètres importants de l'étape de désinfection et conditionnent sa réussite ; la température opératoire est généralement comprise entre 15° C et 25°C, mais dans certains cas peut s'élever jusqu'à 50°C.

Le glutaraldéhyde à 2 % dans l'eau en désinfection manuelle, et à 20-23 % dans l'eau en désinfection automatique, est aujourd'hui la matière active la plus utilisée en Europe. Le temps de désinfection efficace avec ce produit est, en désinfection manuelle, compris entre 20 minutes dans le cas d'un risque infectieux médian, et 1 heure dans le cas d'un risque d'infection élevé. En désinfection par machines, ce temps est compris entre 5 à 20 minutes environ. Cependant, le glutaraldéhyde est toxique, il fixe les protéines et est peu efficace contre les spores. En effet, une solution aqueuse de 2 % en poids de glutaraldéhyde est inefficace à 20°C après 20 minutes, tant sur Bacillus cereus que sur Bacillus subtilis. Il ne devient efficace contre ces souches qu'après au moins 1 heure de trempage.

La demande de brevet européen EP 0 873 687 divulgue une composition comprenant de 0,25 % à 0,75 % en poids d'acide peracétique, de 6 % à 10 % en poids de peroxyde d'hydrogène, de 2 % à 10 % en poids d'acide acétique, de 0,0015 % à 0,75 % en poids d'un oxyde d'amine, et si désiré jusqu'à 0,15 % en poids d'un alcool polyalcoxylé. Cette composition est utilisée pour désinfecter les endoscopes en machine à laver en la diluant entre le 1/3 et le 1/10 et plus particulièrement au 1/5.

C'est pourquoi la demanderesse a recherché un substitut à ce produit ; elle s'est intéressée aux solutions aqueuses d'acide peracétique qui sont connues pour leur efficacité sur l'ensemble des microorganismes, notamment sur les spores, lors qu'elles sont à une concentration suffisante en peracide soit à partir d'environ 5000 ppm pour un abattement de 5 log. et d'environ 2500 ppm pour un abattement de 3 log. en 5 minutes. Cependant à cette concentration, l'acide peracétique devient corrosif pour certains des matériaux constitutifs du matériel médico-chirurgical et notamment pour leurs parties métalliques et il commence aussi à être légèrement irritant sur la peau. On sait aussi, que le peroxyde d'hydrogène seul n'est sporicide qu'à des concentrations minimales de 10 % à 20 % en poids.

La demanderesse a donc cherché à mettre au point un procédé automatique de désinfection des endoscopes en machine, mettant en oeuvre l'acide peracétique, qui permette sur un cycle machine, d'obtenir, un abattement logarithmique de 3 log sur Bacillus cereus, soit une réduction de 10³ spores par cm³, ce résultat étant déterminé par l'analyse normalisée AFNOR T 72 301. Le choix de l'activité sporicide comme indice d'efficacité de l'acide peracétique est dicté par le haut niveau de désinfection exigé ; c'est elle qui généralement requiert la dose la plus élevée en désinfectant, à cause de la présence d'une tunique sporale autour des bactéries augmentant leur résistance. Le choix de Bacillus cereus, comme cible d'étude parmi les souches indiquées dans la norme AFNOR T 72 301, à savoir Bacillus cereus CIP 7803, Bacillus subtilis et Clostridium sporogenes CIP 7939, comme cible de l'activité sporicide de l'acide peracétique, découle du résultat d'un test préliminaire, au cours duquel la demanderesse s'est aperçue que Bacillus cereus était plus facile à détruire que Bacillus subtilis avec du glutaraldéhyde, alors qu'au contraire, Bacillus cereus était plus difficile à détruire que Bacillus subtilis avec de l'acide peracétique.

La demanderesse a trouvé que l'on atteignait le résultat requis, lorsque l'on mettait en oeuvre le procédé selon l'invention, objet de la présente demande de brevet.

L'invention a pour objet un procédé de désinfection d'endoscopes, mis en oeuvre dans des machines à laver et à désinfecter les endoscopes, caractérisé en ce ledit endoscope est mis en contact pendant 5 à 15 minutes dans ladite machine, avec une solution aqueuse obtenue par dilution dans l'eau au 10^{ème}, d'une solution aqueuse initiale comprenant au moins 1 % en poids et au plus 1,5 % en poids d'acide peracétique, au moins 6 % en poids et au plus 8 % en poids de peroxyde d'hydrogène, au moins 6 % en poids et au plus 10 % en poids d'acide acétique.

Par endoscope, on indique dans le procédé tel que défini ci-dessus, notamment les bronchoscopes, les laryngoscopes, les oesophagoscopes, les gastroscopes, les colonoscopes, les rectoscopes, les laparoscopes, les arthroscopes, les cystoscopes, les amnioscopes, les médiastinoscopes, les hystérescopes, les coelioscopes, les sinuscopes, les cholédoscopes transpariétaux, les cholesdoscopes rétrogrades ou les urétéroscopes.

La mise en contact de l'endoscope avec la solution désinfectante, se fait, sur sa surface extérieure, par aspersion et à l'intérieur des canaux par injection de la solution au moyen d'une pompe. Selon les appareils, la solution circule dans un seul circuit ou dans deux circuits indépendants, l'un pour désinfecter l'intérieur de chaque canal, via une pompe individuelle l'autre pour désinfecter l'extérieur de l'endoscope via des bras de lavage.. Selon les appareils et la réglementation en vigueur dans le pays de leur utilisation, le procédé est mis en oeuvre à une température comprise en 15°C et 40°C environ.

L'invention a plus particulièrement pour objet un procédé désinfection tel que défini précédemment, dans lequel l'étape de mise en contact dudit matériel avec ladite solution est précédée d'au moins une étape de nettoyage.

Par étape de nettoyage, on indique une étape dont l'objectif est d'éliminer les salissures et qui va conjuguer l'action physico-chimique de la solution détergente utilisée dans cette étape utilisée et des actions mécaniques effectuées par la machine, telles que le pompage de la solution de lavage dans les canaux de l'endoscope ou l'aspersion.

L'invention a plus particulièrement pour objet un procédé désinfection tel que défini précédemment, dans lequel l'étape de nettoyage dudit matériel, est précédée d'une étape d'une étape de pré-traitement.

Par étape de pré-traitement, on indique une étape dont le but consiste à faciliter les étapes ultérieures en empêchant notamment les salissures de sécher, par exemple en irriguant immédiatement après la fin de leur utilisation, le canal de l'instrument médical en comportant, par une solution détergente et éventuellement bactéricide et/ou en l'immergeant dans ladite solution. Cette étape est effectuée à l'extérieur de la machine.

L'invention a plus particulièrement pour objet, un procédé désinfection tel que défini précédemment, dans lequel l'étape de mise en contact dudit matériel avec ladite solution est suivie d'une étape de rinçage puis, si nécessaire, d'une étape de séchage.

L'étape de rinçage final a pour objectif est d'éliminer tout résidu de produit tout en évitant les re-contaminations ; ceci implique l'utilisation d'une eau adaptée à cette exigence qui, selon le niveau de risque, sera stérile ou filtrée ; le séchage est nécessaire lorsque le dispositif n'est pas immédiatement réutilisé.

II est bien entendu que la présente invention inclut aussi le procédé de désinfection de plusieurs endoscopes lorsque la machine mise en oeuvre le permet.

La solution aqueuse d'acide peracétique mise en contact dans la machine avec l'endoscope à désinfecter, peut comprendre aussi, un ou plusieurs additifs choisis notamment parmi les agents tensioactifs non ioniques, les oxydes d'amines, les inhibiteurs de corrosion, les agents stabilisants ou des acides forts.

Comme agents tensioactifs non ioniques, on désigne dans la présente demande de brevet, ceux décrits dans la demande de brevet européen publiée sous le numéro EP 0 873 687, plus particulièrement, les produits commerciaux suivants : le GENAPOL™ 2822, le GENAPOL™ 2908, le GENAPOL™ 2908D, le GENAPOL™ 2909, le TRITON™ DF12, le TRITON™ DF16, le TRITON™ CF10, le DOWFAX™ 20B102, l'AKYPO™ RO 90, le SYNPERONIC™ LF RA 30 ou le SIMULSOL™ NW 900, l' AKYPO LF2 ou l'AKYPO LF4. Ces produits, disponibles dans le commerce ont la composition chimique suivante :

| **nom commercial** | **composition chimique** |
|---|---|
| GENAPOL™ 2822 | Mélange d'alcools alkoxylés en C₁₀, C₁₂, C₁₄ (5OE, 4OP) |
| GENAPOL™ 2908D | Mélange d'alcools alkoxylés en C₁₁, C₁₃, C₁₅ (6 à 7OE, 3OP) |
| GENAPOL™ 2909 | Mélange d'alcools alkoxylés en C₁₂, C₁₄ (5OE, 3OP) |
| TRITON™ DF12 | Ethers benzyliques d'alcools alkoxylés en C₈, C₁₀ (2OE, 5OP) |
| TRITON™ DF16 | Mélange d'alcools alkoxylés en C₈, C₁₀ (6OE, 3OP) |
| TRITON™ CF10 | Ether benzylique d'alcools éthoxylés en C₈ (16OE) |
| AKYPO™ RO 90 | Mélange d'éthers carboxyliques éthoxylés en C₁₆ C₁₈ (9 OE) |
| DOWFAX™ 20B102 | |
| SIMULSOL™ NW 900 | Mélange d'alcools alkoxylés (x OE, y OP) |
| AKYPO LF2 | Ether carboxylique éthoxylé en C₈ (8 OE) |
| AKYPO LF4 | Mélange d'éthers carboxyliques éthoxylés en C₆, C₈ (7 OE) |

Comme agents tensioactifs non-ioniques, on préfère ceux correspondant à la formule (I) suivante :

R₁-O-[CH(R₂)-CH(R₃)-O]ₙ-R₄ (I)

dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20.

Par radical hydrocarboné linéaire ou ramifié, saturé ou insaturé, comprenant de 5 à 31 atomes de carbone, on désigne pour R₁ dans la formule (I) telle que définie précédemment notamment les radicaux alkyles ou les radicaux alkènyle. R₁ représente plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés. Selon un aspect particulier de la présente invention, R₁ représente un radical choisi de préférence parmi les radicaux alkyles linéaires ou ramifiés comportant de 8 à 18 atomes de carbone.

Par radical alkyle comprenant de 1 à 4 atomes de carbone, on désigne pour R₄ dans la formule (I) telle que définie précédemment, les radicaux méthyle, éthyle, propyle ou butyle, linéaires ou ramifiés.

Le groupe divalent radical -[CH(R₂)-CH(R₃)-O]ₙ- représente, soit une chaîne composée uniquement de groupes éthoxyle (R₂ et R₃ = H), soit une chaîne composée uniquement de groupes propoxyle (R₂ ou R₃ = CH₃), soit une chaîne composée uniquement de groupes butoxyle (R₂ et R₃ = CH₃) soit une chaîne composée d'un mélange de deux sortes ou des trois sortes de groupes énoncés ci-dessus. Dans ce dernier cas, les fragments -CH₂-CH₂-O-, -CH(CH₃)-CH₂-O- et - CH(CH₃)-CH(CH₃)-O-, sont distribués dans ladite chaîne, de façon séquencée ou aléatoire. La solution aqueuse d'acide peracétique mise en contact dans la machine avec l'endoscope à désinfecter dans le procédé objet de la présente invention, peut contenir jusqu'à 0,003% en poids d'au moins un agent tensioactif non ionique.

Comme oxyde d'amine, on désigne dans la présente demande de brevet, ceux décrits dans la demande de brevet européen publiée sous le numéro EP 0 873 687 et notamment ceux correspondant à la formule (II) :

(R₅)(R₆)(R₇) N->O (II)

dans laquelle R₅ représente un radical hydrocarboné linéaire ou ramifié, saturé ou insaturé comprenant de 8 à 18 atomes de carbones, R₆ et R₇ représentent chacun, un radical méthyle et, plus particulièrement, ceux commercialisés sous la marque AROMOX™ et notamment l'AROMOX™ MCD-W qui contient comme matière active, le N-oxyde de cocodiméthylamine. La solution aqueuse d'acide peracétique, mise en contact dans la machine avec l'endoscope à désinfecter dans le procédé objet de la présente invention, peut contenir jusqu'à 0,003 % en poids d'au moins un oxyde d'amine.

La solution aqueuse d'acide peracétique mise en contact dans la machine avec l'endoscope à désinfecter dans le procédé objet de la présente invention, peut contenir jusqu'à 0,4 % en poids d'un inhibiteur de corrosion choisi de préférence entre le dihydrogénophosphate de sodium, NaH₂PO₄ et le phosphate disodique, Na₂HPO₄.

La solution aqueuse d'acide peracétique mise en contact dans la machine avec l'endoscope à désinfecter dans le procédé objet de la présente invention, peut contenir jusqu'à 0,03 % en poids d'un agent stabilisant, qui est de préférence le pyrophosphate de sodium.

L'invention a plus particulièrement pour objet un procédé tel que défini précédemment, caractérisé en ce que la solution aqueuse d'acide peracétique mise en contact dans la machine avec l'endoscope à désinfecter, comprend de 0,10 % à 0,15 % en poids d'acide peracétique, de 0,7 % à 0,8 % en poids de peroxyde d'hydrogène, de 0,9 % à 1,0% en poids d'acide acétique, de 0,001 % à 0,002 % de GENAPOL™ 2908D, de 0,1 % à 0,3 % en poids de dihydrogénophosphate de sodium (12H₂O), de 0,015 % à 0,025 % en poids de pyrophosphate de sodium.

Selon une variante particulière de ce procédé, la solution aqueuse d'acide peracétique mise en contact dans la machine avec l'endoscope à désinfecter, comprend aussi de 0,0015 % à 0,0025 % en poids, de N-oxyde de cocodiméthylamine.

Selon la machine utilisée et le modèle, la dilution dans l'eau de la solution d'origine est de 0 à 200 fois de ladite solution.

Par exemple, les machines de la société LANCER™ procèdent à une dilution de 10 à 30 fois, tandis que les machines des sociétés MEDLORE™ et OLYMPUS™ procèdent à des dilutions de l'ordre de 100 à 150 fois, et que les machines PHACOGENE™ fonctionnent sans dilution.

La durée de vie de la solution diluée est de 1 semaine environ. Si nécessaire, le procédé tel que décrit ci-dessus, comprend aussi l'addition ponctuelle, en continu ou périodique, au cours du cycle de désinfection, d'une quantité supplémentaire de ladite solution aqueuse introduite initialement dans la machine, pour compenser la perte en acide peracétique. On peut aussi désinfecter lesdites machines en faisant tourner à vide, c'est à dire en mettant en oeuvre le procédé tel décrit ci-dessus, en l'absence de matériel à désinfecter. Une telle opération s'effectue à intervalles réguliers, généralement d'une fois par jour à une fois par semaine, selon la fréquence d'utilisation de la machine.

Selon un autre aspect de la présente invention, celle-ci a pour objet une composition prête à l'emploi, particulièrement appropriée à la désinfection des endoscopes en la mettant en oeuvre dans une machine à laver et à désinfecter, comprenant de 1,0 % à 1,5 % en poids d'acide peracétique, de 7 % à 8 % de peroxyde d'hydrogène, de 9 % à 10 % d'acide acétique, de 0,010 % à 0,020 % de GENAPOL™ 2908D, de 1 % à 3 % de dihydrogénophosphate de sodium (12 H₂O), de 0,15 % à 0,25 % de pyrophosphate de sodium et éventuellement contenant aussi 0,015 % à 0,025 % de N-oxyde de cocodiméthylamine.

### Exemple

### Cas des machines à laver et à désinfecter

Différents essais ont validé la bonne efficacité contre les bactéries, moisissures, virus et spores bactériens, dans une machine à laver et à désinfecter les endoscopes, commercialisée par la société LANCER, de la désinfection d'un endoscope par sa mise en contact, pendant une phase désinfection de 10 minutes, avec une solution désinfectante comprenant environ 0,11 % en poids d'acide peracétique, et environ 0,72 % de peroxyde d'hydrogène, la machine ayant procédé préalablement à une dilution au 10ème d'une solution contenant de 1,0 % à 1,5 % en poids d'acide peracétique, de 7 % à 8 % en poids de peroxyde d'hydrogène, de 9 % à 10 % en poids d'acide acétique, de 0,010 % à 0,020 % en poids de GENAPOL™ 2908D, de 1 % à 3 % en poids de dihydrogénophosphate de sodium (12H₂O), de 0,15 % à 0,25 % en poids de pyrophosphate de sodium, de 0,015 % à 0,025 % en poids de N-oxyde de cocodiméthylamine.

## Revendications

1. Procédé de désinfection d'un endoscope, **caractérisé en ce que** ledit endoscope est mis en contact pendant 5 à 15 minutes, dans une machine à laver et à désinfecter les endoscopes, avec une solution aqueuse, obtenue par dilution dans l'eau au 10^{ème}, d'une solution aqueuse initiale comprenant au moins 1 % en poids et au plus 1,5 % en poids d'acide peracétique, au moins 6% en poids et au plus 8% en poids de peroxyde d'hydrogène, au moins 6 % en poids et au plus 10 % en poids d'acide acétique.

2. Procédé tel que défini à la revendication 1, dans lequel l'étape de mise en contact de l'endoscope avec ladite solution, est précédée dans ladite machine d'au moins une étape de nettoyage.

3. Procédé tel que défini à la revendication 2, dans lequel, l'étape de nettoyage dudit matériel, est précédée d'une étape de pré-traitement.

4. Procédé tel que défini à l'une des revendications 1 à 3, dans lequel l'étape de mise en contact de l'endoscope avec ladite solution, est suivie d'une étape de rinçage.

5. Procédé tel que défini à la revendication 4, dans lequel l'étape de rinçage, est suivie d'une étape de séchage.

6. Procédé tel que défini à l'une des revendications 1 à 5, **caractérisé en ce que** la solution aqueuse d'acide peracétique mise en contact dans la machine avec l'endoscope à désinfecter, est obtenue par dilution dans l'eau au 10^{ème} d'une solution aqueuse comprenant de 1,0 % à 1,5 % en poids d'acide peracétique, de 7 % à 8 % en poids de peroxyde d'hydrogène, de 9 % à 10 % en poids d'acide acétique, de 0,010 % à 0,020 % en poids de GENAPOL™ 2908D, de 1 % à 3 % en poids de dihydrogénophosphate de sodium (12 H₂O), et de 0,15 % à 0,25 % en poids de pyrophosphate de sodium.

7. Procédé tel que défini à la revendication 6, **caractérisé en ce que** la solution aqueuse d'acide peracétique diluée au 10^{ème} contient aussi de 0,015 % à 0,025 % en poids, de N-oxyde de cocodiméthylamine.

8. Composition sous forme d'une solution aqueuse comprenant de 1,0 % à 1,5 % en poids d'acide peracétique, de 7 % à 8 % en poids de peroxyde d'hydrogène, de 9 % à 10 % en poids d'acide acétique, de 0,010 % à 0,020 % en poids de GENAPOL™ 2908D, de 1 % à 3 % en poids de dihydrogénophosphate de sodium (12H₂O) et de 0,15 % à 0,25 % en poids de pyrophosphate de sodium.

9. Composition telle que définie à la revendication 8, comprenant en outre de 0,015 % à 0,025 % en poids, de N-oxyde de cocodiméthylamine.

## Patentansprüche

1. Verfahren zur Desinfektion eines Endoskops, **dadurch gekennzeichnet, dass** das Endoskop für 5 bis 15 Minuten in einer Maschine zum Waschen und Desinfizieren von Endoskopen mit einer wässrigen Lösung in Kontakt gebracht wird, die erhalten wird durch Verdünnen einer wässrigen Ausgangslösung, die mindestens 1 Gew.-% und höchstens 1,5 Gew.-% Peressigsäure, mindestens 6 Gew.-% und höchstens 8 Gew.-% Wasserstoffperoxid, mindestens 6 Gew.-% und höchstens 10 Gew.-% Essigsäure umfasst, in Wasser auf 1/10tel.

2. Verfahren nach Anspruch 1, wobei dem Schritt des In-Kontakt-Bringens des Endoskops mit der Lösung in der Maschine mindestens ein Putzschritt vorausgeht.

3. Verfahren nach Anspruch 2, wobei dem Schritt des Putzens des Materials ein Vorbehandlungsschritt vorausgeht.

4. Verfahren nach einem der Ansprüche 1 bis 3, wobei auf den Schritt des In-Kontakt-Bringens des Endoskops mit der Lösung ein Spülschritt folgt.

5. Verfahren nach Anspruch 4, wobei auf den Spülschritt ein Trocknungsschritt folgt.

6. Verfahren nach einem der Ansprüche 1 bis 5, **dadurch gekennzeichnet, dass** die in der Maschine mit dem zu desinfizierenden Endoskop in Kontakt gebrachte wässrige Peressigsäurelösung erhalten wird durch Verdünnung einer wässrigen Lösung, die 1,0 Gew.-% bis 1,5 Gew.-% Peressigsäure, 7 Gew.-% bis 8 Gew.-% Wasserstoffperoxid, 9 Gew.-% bis 10 Gew.-% Essigsäure, 0,010 Gew.-% bis 0,020 Gew.-% GENAPOL™ 2908D, 1 Gew.-% bis 3 Gew.-% Natriumdihydrogenphosphat (12 H₂O) und 0,15 Gew.-% bis 0,25 Gew.-% Natriumpyrophosphat umfasst, in Wasser auf ein 1/10tel.

7. Verfahren nach Anspruch 6, **dadurch gekennzeichnet, dass** die auf ein 1/10tel verdünnte wässrige Peressigsäurelösung ferner 0,015 Gew.-% bis 0,025 Gew.-% Cocodimethylamin-N-oxid umfasst.

8. Zusammensetzung in Form einer wässrigen Lösung, die 1,0 Gew.-% bis 1,5 Gew.-% Peressigsäure, 7 Gew.-% bis 8 Gew.-% Wasserstoffperoxid, 9 Gew.-% bis 10 Gew.-% Essigsäure, 0,010 Gew.-% bis 0,020 Gew.-% GENAPOL™ 2908D, 1 Gew.-% bis 3 Gew.-% Natriumdihydrogenphosphat (12 H₂O) und 0,15 Gew.-% bis 0,25 Gew.-% Natriumpyrophosphat umfasst.

9. Zusammensetzung nach Anspruch 8, die ferner 0,015 Gew.-% bis 0,025 Gew.-% Cocodimethylamin-N-oxid umfasst.

## Claims

1. Method of disinfecting an endoscope, **characterized in that** the said endoscope is brought into contact, for 5 to 15 minutes, in a machine for washing and disinfecting endoscopes, with an aqueous solution, obtained by diluting tenfold in water an initial aqueous solution comprising at least 1% by weight and at most 1.5% by weight of peracetic acid, at least 6% by weight and at most 8% by weight of hydrogen peroxide, and at least 6% by weight and at most 10% by weight of acetic acid.

2. Method as defined in Claim 1, in which the step of bringing of the endoscope into contact with the said solution is preceded in the said machine by at least one cleaning step.

3. Method as defined in Claim 1, in which the step of cleaning the said equipment is preceded by a pretreatment step.

4. Method as defined in one of Claims 1 to 3, in which the step of bringing the endoscope into contact with the said solution is followed by a rinsing step.

5. Method as defined in Claim 4, in which the rinsing step is followed by a drying step.

6. Method as defined in one of Claims 1 to 5, **characterized in that** the aqueous solution of peracetic acid brought into contact, in the machine, with the endoscope to be disinfected is obtained by diluting tenfold in water an aqueous solution comprising from 1.0% to 1.5% by weight of peracetic acid, from 7% to 8% by weight of hydrogen peroxide, from 9% to 10% by weight of acetic acid, from 0.010% to 0.020% by weight of GENAPOL™ 2908D, from 1% to 3% by weight of sodium dihydrogen phosphate (12 H₂O) and from 0.15% to 0.25% by weight of sodium pyrophosphate.

7. Method as defined in Claim 6, **characterized in that** the new tenfold diluted aqueous solution of peracetic acid contains also from 0.015% to 0.025% by weight of cocodimethylamine N-oxide.

8. Composition in the form of an aqueous solution comprising from 1.0% to 1.5% by weight of peracetic acid, from 7% to 8% by weight of hydrogen peroxide, from 9% to 10% by weight of acetic acid, from 0.010% to 0.020% by weight of GENAPOL™ 2908D, from 1% to 3% by weight of sodium dihydrogen phosphate (12H₂O) and from 0.15% to 0.25% by weight of sodium pyrophosphate.

9. Composition as defined in Claim 8, additionally comprising from 0.015% to 0.025% by weight of cocodimethylamine N-oxide.
